# FASCICULE DE BREVET EUROPEEN

(11) **EP 2 555 714 B1**
(45) Date de publication et mention de la délivrance du brevet: **01.01.2014**
(21) Numéro de dépôt: 11730347.9
(22) Date de dépôt: 05.04.2011
(51) Int. Cl.: A61F 2/44, A61F 2/46

(54) **SYSTEME POUR REMPLACER UN DISQUE INTERVERTEBRAL**
SYSTEM FÜR BANDSCHEIBENERSATZ
SYSTEM FOR REPLACING AN INTERVERTEBRAL DISC

(30) Priorité: 06.04.2010 FR 1001398
(43) Date de publication de la demande: 13.02.2013
(73) Titulaire: Razian, Hassan, 94240 l'Hay les Roses (FR)
(72) Inventeur: RAZIAN, Sam, F-94240 L'Hay les Roses (FR)
(74) Mandataire: Flavenot, Bernard
(86) Numéro de dépôt international: PCT/FR2011/000197
(87) Numéro de publication internationale: WO 2011/124787

(56) Documents cités:
- WO-A1-02/17823
- WO-A2-2008/016598
- FR-A1- 2 861 582
- US-A1- 2008 221 694

## Description

La présente invention concerne les systèmes pour remplacer un disque intervertébral.

Il est connu que, selon les traumatismes subis par une colonne vertébrale, les praticiens sont amenés à effectuer deux sortes d'intervention chirurgicale.

Quand le traumatisme ne concerne qu'un disque intervertébral et que les deux vertèbres consécutives encadrant ce disque sont en bon état, le praticien peut opter pour le remplacement du disque par une prothèse dont la fonctionnalité est sensiblement équivalente à celle du disque originel, c'est-à-dire laissant aux deux vertèbres la possibilité de se déplacer l'une par rapport à l'autre dans des mouvements de rotation et/ou de translation.

En revanche, quand le traumatisme est plus important, le praticien peut choisir de solidariser les deux vertèbres consécutives par ostéosynthèse. Pour ce faire, le disque intervertébral est partiellement ou en totalité détruit et remplacé par une cage intersomatique associée généralement à un élément favorisant l'ostéosynthèse, par exemple un greffon osseux.

Ces cages intersomatiques sont implantables essentiellement par deux voies, l'une dite "antérieure", l'autre dite "postérieure", chacune de ces voies ayant ses avantages et ses inconvénients.

Il semblerait cependant évident que l'implantation par la voie postérieure soit la plus normale car le chemin utilisé par le praticien pour l'intervention chirurgicale est relativement court. Mais elle est très délicate car ce chemin passe très près d'éléments essentiels pour la vie du patient, à savoir la moelle épinière et les deux nerfs qui commandent notamment les mouvements du patient.

C'est pour cette raison que certains praticiens optent parfois pour la voie dite "antérieure" qui implique un chemin d'intervention beaucoup plus long, mais en théorie moins dangereux pour le patient. A contrario, le temps d'intervention est plus long que celui par la voie postérieure, ce qui peut présenter un inconvénient pour la santé et la récupération du patient.

Aussi a-t-il été réalisé des nombreux systèmes pour remplacer un disque entre deux vertèbres consécutives, qui permet une implantation par la voie notamment postérieure.

De tels systèmes sont par exemple décrits et illustrés dans le US/20050027360, le WO20081016598 et le WO/0044288. Ces systèmes sont malgré tout relativement complexes, encombrants et difficiles à utiliser.

Le Demandeur a, lui aussi, réalisé de tels systèmes, par exemple celui décrit dans le FR-A- 2914842.

La présente invention a cependant pour but de réaliser un système pour remplacer un disque entre deux vertèbres consécutives, qui permet une implantation par la voie postérieure, qui soit d'une structure plus simple, et donc moins encombrante et plus facile à utiliser que celle des systèmes de l'art antérieur en ce domaine, par exemple ceux définis dans les documents précités.

Plus précisément, la présente invention a pour objet un système pour remplacer un disque entre deux vertèbres consécutives, telle que définie dans la revendication 1 annexée dont le préambule contient les caractéristiques du dispositif divulgué dans le WO2008/016598 cité ci-dessus.

D'autres caractéristiques et avantages de l'invention apparaîtront au cours de la description suivante donnée en regard des dessins annexés à titre illustratif mais nullement limitatif, dans lesquels :

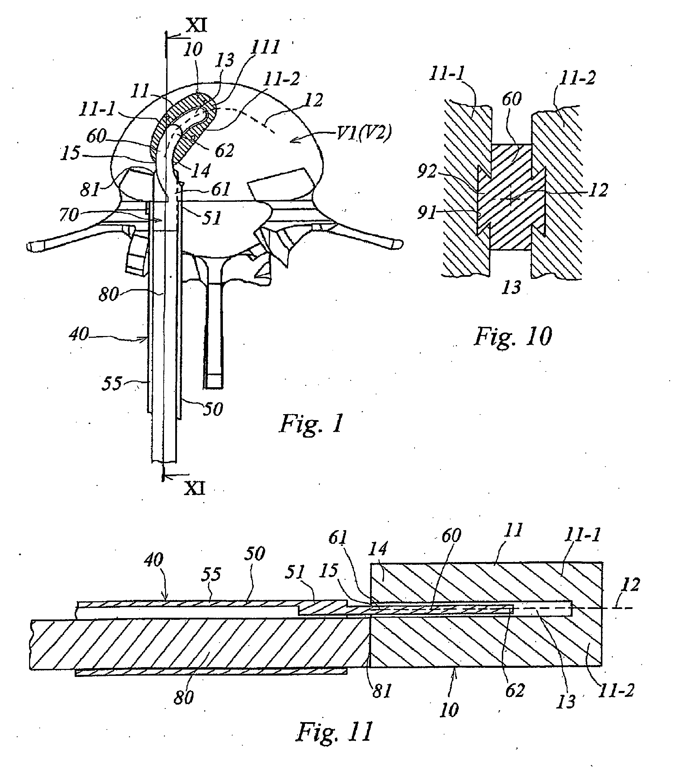

La figure 1 représente, de façon très schématique, un mode de réalisation du système selon l'invention pour remplacer un disque entre deux vertèbres consécutives,

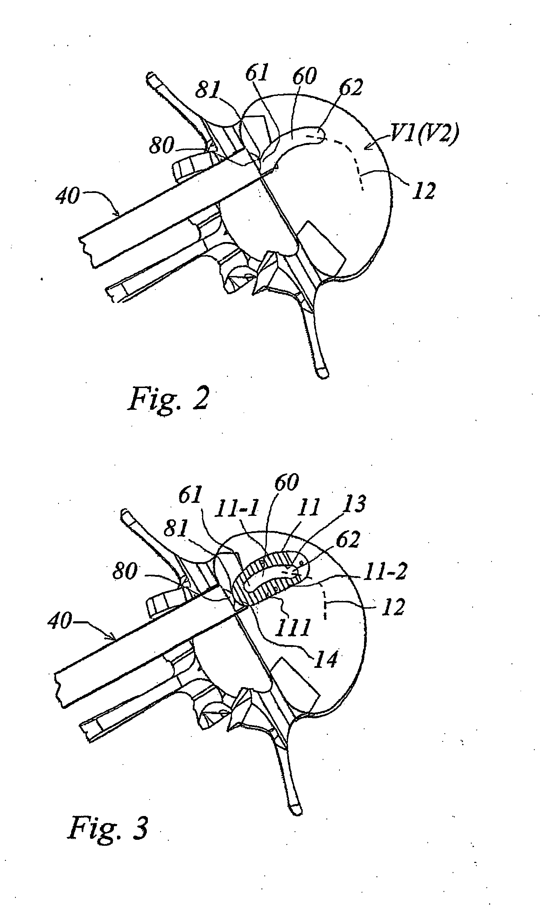

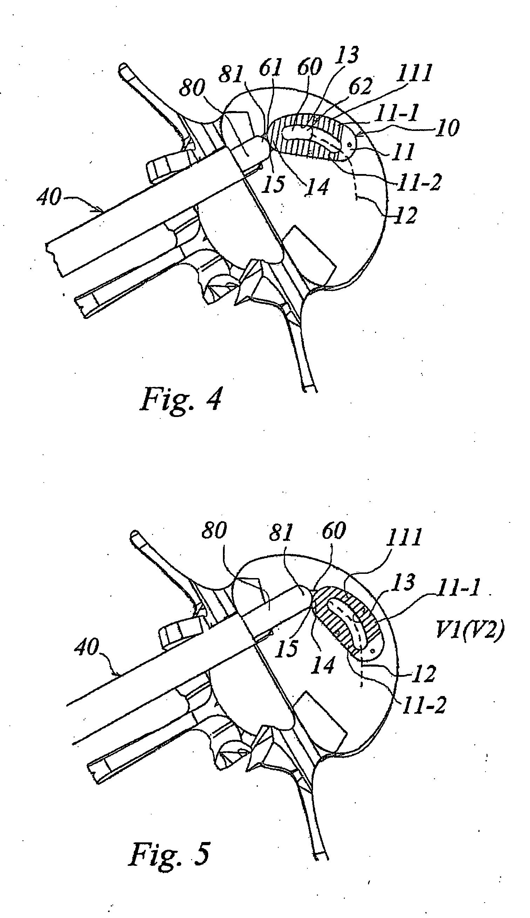

Les figures 2 à 5 représentent différentes configurations du système selon l'invention dans le mode de réalisation selon la figure 1, la figure 2 représentant le système sans son élément "cage", la figure 3 représentant le système dans sa configuration initiale, la cage étant déjà interposée entre deux vertèbres, la figure 4 représentant le système dans une configuration intermédiaire, la cage étant positionnée dans un état proche de sa position finale, et la figure 5 représentant le système avec la cage dans son état final,

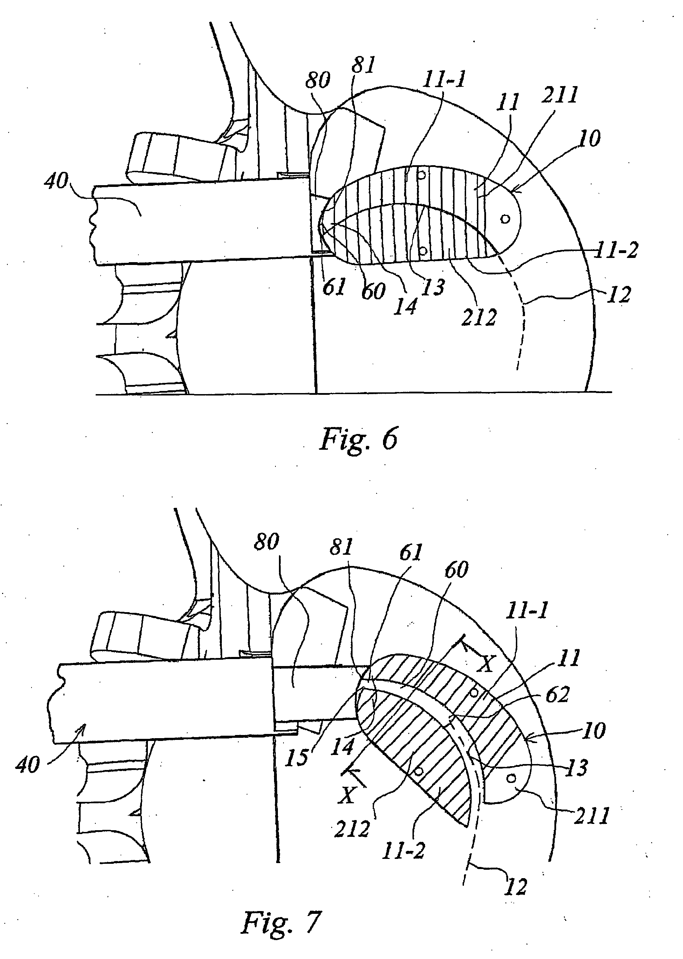

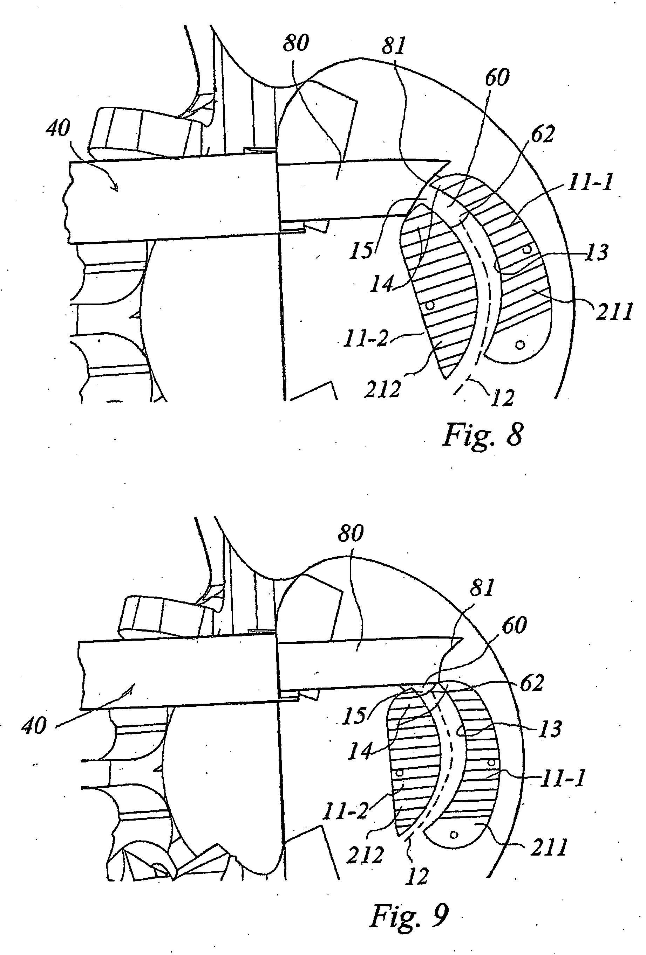

Les figures 6 à 9 représentent différentes configurations du mode de réalisation du système selon la figure 1 dans son utilisation pour l'implantation d'une cage ayant une autre structure que celle selon les figures 1 à 5, la figure 6 représentant le système dans sa configuration initiale, la cage étant déjà interposée entre les deux vertèbres en situation initiale, les figures 7 et 8 représentant le système respectivement dans deux configurations intermédiaires, et la figure 9 représentant le système avec la cage en situation finale, et
Les figures 10 et 11 représentent, de façon schématique, respectivement deux coupes du système selon l'invention permettant d'expliciter sa structure, la figure 10 représentant une coupe partielle du système référencée X-X sur la figure 7 et la figure 11 représentant une coupe du système référencée XI-XI sur la figure 1.

II est précisé que les figures représentent essentiellement deux modes de réalisation de l'objet selon l'invention, mais qu'il peut exister d'autres modes de réalisation qui répondent à la définition de cette invention.

En référence plus particulièrement à la figure 1, la présente invention se rapporte à un système pour remplacer un disque entre deux vertèbres consécutives V1, V2 (une seule apparaissant sur les figures), comprenant au moins une cage intervertébrale 10 et un guide d'introduction 40 pour introduire cette cage intervertébrale entre les deux vertèbres.

Par cage intervertébrale 10 au sens de la présente invention, on entend une cage intervertébrale proprement dite selon la terminaison des hommes du métier, ainsi que tout corps apte à être introduit entre deux vertèbres, qu'il soit en une seule partie ou en plusieurs, quelles que soient sa ou ses fonctions, par exemple une prothèse discale. L'expression "cage intervertébrale" n'est utilisée ici que pour simplifier et faciliter la compréhension de l'invention et de sa description.

Selon une caractéristique essentielle de l'invention, la cage intervertébrale 10 est constituée d'un corps oblong 11 délimité entre deux faces opposées sensiblement planes, le corps oblong comportant au moins deux parties 11-1, 11-2 séparées par un espace 13, cet espace étant défini suivant un axe courbe 12 contenu dans un plan médian sensiblement parallèle aux plans des faces opposées et débouchant par une ouverture 15 à une première extrémité 14 du corps oblong 11 entre les deux parties 11-1, 11-2 du corps oblong.

Selon une autre caractéristique de l'invention, le guide d'introduction 40 est constitué d'au moins une tige de guidage 50, dont un exemple avantageux de mode de réalisation sera décrit ci-après, et une âme longitudinale courbe 60 dont les sections transversales sont sensiblement complémentaires de celles de l'espace 13, de façon à permettre un coulissement respectif entre l'âme courbe et les deux parties 11-1, 11-2 du corps oblong 11.

Deux exemples de réalisation de cette âme courbe 60 seront donnés ci-après en regard respectivement des figures 2 à 5, et des figures 6 à 9.

Sont aussi prévus des moyens 70 pour monter une première extrémité 61 de l'âme courbe 60 en coopération avec une première extrémité 51 de la tige de guidage 50 de façon que cette âme courbe soit en saillie par rapport à cette première extrémité 51 de la tige de guidage, et une tige poussoir 80 montée en déplacement par rapport à la tige de guidage 50 de façon qu'elle soit apte à venir au contact, par une 81 de ses extrémités, contre la première extrémité 14 du corps oblong 11 pour pousser ce corps oblong en translation par rapport à l'âme courbe 60.

Selon une première réalisation possible, le corps oblong 11 est constitué d'une pièce unique 111 et l'espace 13 est constitué d'une percée réalisée dans la pièce unique sensiblement suivant l'axe courbe 12 défini précédemment.

Il est aussi avantageux que cette percée soit ouverte sur les deux faces opposées de la cage intervertébrale 10 sensiblement planes de façon à pouvoir éventuellement y placer un greffon osseux ou analogue.

De façon avantageuse, comme illustré, la pièce unique 111 a sensiblement la forme d'un "haricot" ou assimilé, mais à faces opposées sensiblement planes.

Dans ce cas, selon les figures 1 à 5, toutes les sections transversales de l'âme courbe 60 définies dans des plans perpendiculaires à l'axe courbe 12 sont sensiblement identiques entre elles.

Selon une première possibilité, pour empêcher que la pièce unique 111 ne puisse pivoter au moins partiellement autour de l'âme courbe 60, mais en revanche pour permettre à cette pièce unique 111 de coulisser par rapport à cette âme courbe, il est très préférable que les sections transversales de cette âme courbe 60, définies dans des plans perpendiculaires à l'axe courbe 12, soient de forme polygonale. Comme exemple, dans le mode de réalisation illustré sur la figure 10, la section transversale de l'âme courbe est de forme rectangulaire, mais d'autres formes sont possibles.

En se référant maintenant plus particulièrement au mode de réalisation selon les figures 6 à 10, les deux parties 11-1, 11-2 du corps oblong 11 ne forment pas une pièce unique, mais sont constituées de deux pièces 211, 212 séparées l'une de l'autre.

Dans ce cas, dans le but explicité ci-après, il est possible, et même avantageux, que les sections transversales de l'âme courbe 60 définies dans des plans perpendiculaires à l'axe courbe 12 aient des valeurs croissantes de façon sensiblement continue à partir de sa première extrémité 61 jusqu'à sa seconde extrémité libre 62 opposée à la première.

Cependant, bien que le mode de réalisation décrit ci-dessus soit souvent très avantageux, il est possible que les sections transversales de l'âme courbe 60 définies dans des plans perpendiculaires à l'axe courbe 12 soient sensiblement identiques entre elles.

Il a été mentionné que la section de l'âme courbe 60 devait être avantageusement polygonale. Cependant, elle peut être par exemple circulaire, mais dans ce cas on lui adjoindra, comme plus particulièrement visible sur la figure 10, au moins un couple, de préférence deux couples opposés, comprenant une rainure 91 réalisée dans l'un des deux éléments suivants : l'âme courbe 60 et l'une des parties 11-1, 11-2 du corps oblong 11, et une nervure 92 complémentaire de la rainure 91 et réalisée dans l'autre des deux éléments, de façon que la nervure 92 soit apte à coulisser dans la rainure 91.

Sur la figure 10, la rainure 91 est réalisée dans l'une des parties 11-1, 11-2 du corps oblong 11, tandis que la nervure 92 est réalisée sur l'âme courbe 60.

De façon avantageuse, comme dans le mode de réalisation illustré sur cette figure 10, la rainure 91 et la nervure 92 coopèrent selon un montage en queue d'aronde bien connu en lui-même.

Comme mentionné précédemment, le système comporte une tige de guidage 50. Comme illustré et plus particulièrement visible sur la figure 11, cette tige de guidage 50 est constituée d'un tube creux 55 et la tige poussoir 80 est alors montée coulissante dans ce tube creux. Les moyens 70 pour monter la première extrémité 61 de l'âme courbe 60 en coopération avec la première extrémité 51 de la tige de guidage 50 sont alors constitués par des moyens de fixation de la première extrémité 61 de l'âme courbe 60 sur la paroi du tube creux et en continuité de celle-ci.

Le tube creux 55 et l'âme courbe 60 peuvent être réalisés en deux pièces séparées et solidarisées ensemble, ou d'une seule pièce réalisée à partir d'un tube creux unique. Ces réalisations sont du domaine de l'homme du métier ne seront pas plus amplement décrites ici par l'unique souci de simplifier la présente description.

Le système selon l'invention et selon les deux modes de réalisation respectivement figures 1 à 5 et figures 6 à 10 s'utilise de la façon suivante.

De façon connue, le Praticien pratique d'abord une incision dans le dos du patient pour créer un chemin d'introduction qui passe notamment entre les deux nerfs dont l'aspect vital est bien connu des Praticiens.

PREMIER MODE DE REALISATION DU SYSTEME. FIGURES 1 à 5 et 11 : le Praticien introduit, par cette incision, le guide d'introduction 40 avec l'âme courbe 60 déjà introduite entièrement dans la percée 13 du corps oblong 11. De cette façon, la cage 10 est presque totalement en continuité du tube creux 55, comme illustré sur la figure 3. Cette configuration permet aisément l'introduction de la cage 10 entre les deux vertèbres V1, V2 en passant entre les deux nerfs.

Quand la cage est positionnée comme illustré sur la figure 3, le Praticien pousse la tige poussoir 80 dans le tube creux 55 de façon que son extrémité 81 vienne au contact de l'extrémité 14 du corps oblong 11. Il continue à pousser sur la tige poussoir 80 et, sous l'effet de cette poussée, le corps oblong 11 se déplace par rapport à l'âme courbe 60 en suivant l'axe courbe 12 jusqu'à sa position finale (figure 5), en remplacement du disque intervertébral entre les deux vertèbres V1, V2, c'est-à-dire en lui ayant fait subir une rotation d'environ quatre-vingt-dix de degrés par rapport à sa direction d'introduction.

Lorsque la cage 10 arrive dans sa position finale, l'âme courbe 60 est totalement sortie de la percée 13 et le guide d'introduction 40 peut être retiré en sens inverse du chemin suivi pour son introduction.

SECOND MODE DE REALISATION DU SYSTEME, FIGURES 6 à 10 et 11 : le Praticien introduit, par l'incision pratiquée précédemment, le guide d'introduction 40 avec l'âme courbe 60 introduite entièrement dans la percée 13 du corps oblong 11. De cette façon, la cage 10 est presque totalement en continuité du tube creux 55, comme illustré sur la figure 6, et cette configuration permet aisément l'introduction de la cage 10 entre les deux vertèbres V1, V2 en passant entre les deux nerfs et autres organes vitaux.

Cependant, dans ce cas, le corps oblong 11 étant constitué de deux pièces 211, 212 séparées l'une de l'autre, celles-ci sont montées en coopération de façon que les deux nervures 92 réalisées sur l'âme courbe 60 soient montées coulissantes dans les deux rainures 91 réalisées respectivement sur les deux pièces 211, 212. De cette façon les trois éléments : l'âme courbe 60 et les deux pièces 211, 212 sont bien maintenus les uns par rapport aux autres.

Le positionnement de la cage entre les deux vertèbres V1, V2 s'effectue comme décrit ci-dessus en regard des figures 1 à 5 et 11 pour le premier mode de réalisation du système. Cependant, comme dans ce cas les deux pièces 211, 212 sont séparées l'une de l'autre et les sections de l'âme courbe 60 sont croissantes comme défini auparavant, en poussant ces deux pièces 211, 212 par rapport à l'âme courbe 60, au fur et à mesure qu'elles se déplacent, elles s'écartent progressivement l'une de l'autre, comme illustré par les quatre figures 6 à 9, jusqu'à ce qu'elles prennent leur position définitive, figure 9.

Cette seconde réalisation permet, en quelque sorte, de positionner simultanément deux demi-cages 211, 212 éloignées l'une de l'autre pour mieux remplir l'espace intervertébral en remplacement du disque intervertébral originel, avec la possibilité de placer, dans cet espace 13, par exemple un greffon osseux ou analogue.

Bien entendu, il est aussi possible d'utiliser le système selon l'invention avec une cage en deux parties 211, 212 séparées l'une de l'autre et une âme courbe 60 ayant une section uniforme sur toute sa longueur, et non croissante comme dans le mode de réalisation ci-dessus.

## Revendications

1. Système pour remplacer un disque entre deux vertèbres consécutives (V1, V2) comprenant au moins une cage intervertébrale (10) et un guide d'introduction (40) pour introduire ladite cage intervertébrale entre les deux vertèbres, ladite cage intervertébrale (10) étant constituée d'un corps oblong (11) délimité entre deux faces opposées sensiblement planes, ledit corps oblong comportant au moins deux parties (11-1, 11-2) séparées par un espace (13), ledit espace étant défini suivant un axe courbe (12) contenu dans un plan médian sensiblement parallèle aux plans des faces opposées et débouchant par une ouverture (15) à une première extrémité (14) du corps oblong (11) entre les deux dites parties (11-1, 11-2) du dit corps oblong, et le guide d'introduction (40) étant constitué d'au moins une tige de guidage (50),
**caractérisé par le fait que** ledit guide d'introduction est constitué en outre :
- d'une âme longitudinale courbe (60) dont les sections transversales sont sensiblement complémentaires de celles du dit espace (13), de façon à permettre un coulissement respectif entre ladite âme courbe et les deux parties (11-1, 11-2) du dit corps oblong (11),
- de moyens (70) pour monter une première extrémité (61) de ladite âme courbe (60) en coopération avec une première extrémité (51) de ladite tige de guidage (50) de façon que ladite âme courbe (60) soit en saillie par rapport à cette première extrémité (51) de ladite tige de guidage (50), et
- d'une tige poussoir (80) montée en déplacement par rapport à ladite tige de guidage (50) de façon qu'elle soit apte à venir au contact, par une (81) de ses extrémités, contre la première extrémité (14) du corps oblong (11) pour pousser ledit corps oblong en translation par rapport à ladite âme courbe (60).

2. Système selon la revendication 1, **caractérisé par le fait que** ledit corps oblong (11) est constitué d'une pièce unique (111) et que ledit espace (13) est constituée d'une percée réalisée dans ladite pièce unique sensiblement suivant ledit axe courbe (12).

3. Système selon la revendication 2, **caractérisé par le fait que** la pièce unique (111) a sensiblement la forme d'un "haricot" à faces opposées sensiblement planes.

4. Système selon l'une des revendications 2 et 3, **caractérisé par le fait que** toutes les sections transversales de ladite âme courbe (60) définies dans des plans perpendiculaires au dit axe courbe (12) sont sensiblement identiques entre elles.

5. Système selon la revendication 4, **caractérisé par le fait que** les sections transversales de ladite âme courbe (60) définies dans des plans perpendiculaires au dit axe courbe (12) sont de forme polygonale.

6. Système selon la revendication 1, **caractérisé par le fait que** les deux parties (11-1, 11-2) du dit corps oblong (11) sont constituées de deux pièces (211, 212) séparées l'une de l'autre.

7. Système selon la revendication 6, **caractérisé par le fait que** les sections transversales de ladite âme courbe (60) définies dans des plans perpendiculaires au dit axe courbe (12) ont des valeurs croissantes de façon sensiblement continue à partir de sa première extrémité (61) jusqu'à son autre seconde extrémité libre (62) opposée à la première.

8. Système selon la revendication 6, **caractérisé par le fait que** toutes les sections transversales de ladite âme courbe (60) définies dans des plans perpendiculaires au dit axe courbe (12) sont sensiblement identiques entre elles.

9. Système selon l'une des revendications précédentes, **caractérisé par le fait qu'**il comporte en outre au moins un couple comprenant une rainure (91) réalisée dans l'un des deux éléments suivants : l'âme courbe (60) et l'une des parties (11-1, 11-2) du dit corps oblong (11), et une nervure (92) complémentaire de ladite rainure (91), ladite nervure étant réalisée dans l'autre des deux éléments de façon que ladite nervure (92) soit apte à coulisser dans ladite rainure (91).

10. Système selon la revendication 9, **caractérisé par le fait que** ladite rainure (91) et ladite nervure (92) coopèrent selon un montage en queue d'aronde.

11. Système selon l'une des revendications précédentes, **caractérisé par le fait que** ladite tige de guidage (50) est constituée d'un tube creux (55), que la tige poussoir (80) est montée coulissante dans ledit tube creux, et que les moyens (70) pour monter la première extrémité (61) de ladite âme courbe (60) en coopération avec la première extrémité (51) de ladite tige de guidage (50) sont constitués par des moyens de fixation de la première extrémité (61) de ladite âme courbe (60) sur la paroi du dit tube creux et en continuité de celle-ci.

## Patentansprüche

1. System zum Austauschen einer Scheibe zwischen zwei aufeinander folgenden Wirbeln (V1, V2), das wenigstens einen Zwischenwirbelkäfig (10) und eine Einführungsführung (40) zum Einführen des Zwischenwirbelkäfigs zwischen die zwei Wirbel umfasst, wobei der Zwischenwirbelkäfig (10) aus einem länglichen Körper (11) gebildet ist, der zwischen zwei im Wesentlichen ebenen gegenüberliegenden Flächen begrenzt ist, wobei der längliche Körper wenigstens zwei durch einen Zwischenraum (13) getrennte Teile (11-1, 11-2) aufweist, wobei der Zwischenraum längs einer ununterbrochenen gekrümmten Achse (12) in einer Medianebene, die zu den Ebenen der gegenüberliegenden Flächen im Wesentlichen parallel ist, definiert ist und durch eine Öffnung (15) in einem ersten Ende (14) des länglichen Körpers (11) zwischen den zwei genannten Teilen (11-1, 11-2) des länglichen Körpers mündet, wobei die Einführungsführung (40) aus wenigstens einem Führungsstift (50) gebildet ist,
**dadurch gekennzeichnet, dass** die Einführungsführung außerdem gebildet ist:
- aus einer longitudinalen gekrümmten Seele (60), deren Querschnitte zu jenen des Zwischenraums (13) im Wesentlichen komplementär sind, derart, dass ein jeweiliges Gleiten zwischen der gekrümmten Seele und den zwei Teilen (11-1,11-2) des länglichen Körpers (11) möglich ist,
- aus Mitteln (70), um ein erstes Ende (61) der gekrümmten Seele (60) in Zusammenwirkung mit einem ersten Ende (51) des Führungsstifts (50) zu montieren, derart, dass die gekrümmte Seele (60) in Bezug auf dieses erste Ende (51) des Führungsstifts (50) vorsteht, und
- aus einem Druckstift (80), der verlagerbar in Bezug auf den Führungsstift (50) montiert ist, derart, dass er mit einem (81) seiner Enden mit dem ersten Ende (14) des länglichen Körpers (11) in Kontakt gelangen kann, um den länglichen Körper translatorisch in Bezug auf die gekrümmte Seele (60) zu schieben.

2. System nach Anspruch 1, **dadurch gekennzeichnet, dass** der längliche Körper (11) aus einem einzigen Teil (111) gebildet ist und dass der Zwischenraum (13) aus einem Loch gebildet ist, das in dem einzigen Teil im Wesentlichen längs der gekrümmten Achse (12) verwirklicht ist.

3. System nach Anspruch 2, **dadurch gekennzeichnet, dass** das einzige Teil (111) im Wesentlichen die Form einer "Bohne" mit im Wesentlichen ebenen gegenüberliegenden Flächen hat.

4. System nach einem der Ansprüche 2 und 3, **dadurch gekennzeichnet, dass** sämtliche Querschnitte der gekrümmten Seele (60), die in den zu der gekrümmten Achse (12) senkrechten Ebenen definiert sind, zueinander im Wesentlichen gleich sind.

5. System nach Anspruch 4, **dadurch gekennzeichnet, dass** die Querschnitte der gekrümmten Seele (60), die in zu der gekrümmten Achse (12) senkrechten Ebenen definiert sind, polygonförmig sind.

6. System nach Anspruch 1, **dadurch gekennzeichnet, dass** die zwei Teile (11-1, 11-2) des länglichen Körpers (11) aus zwei voneinander getrennten Teilen (211, 212) gebildet sind.

7. System nach Anspruch 6, **dadurch gekennzeichnet, dass** die Querschnitte der gekrümmten Seele (60), die in zu der gekrümmten Achse (12) senkrechten Ebenen definiert sind, ausgehend von ihrem ersten Ende (61) im Wesentlichen kontinuierlich zunehmende Werte bis zu ihrem anderen, freien zweiten Ende (62) gegenüber dem ersten Ende haben.

8. System nach Anspruch 6, **dadurch gekennzeichnet, dass** die Querschnitte der gekrümmten Seele (60), die in zu der gekrümmten Achse (12) senkrechten Ebenen definiert sind, im Wesentlichen einander gleich sind.

9. System nach einem der vorhergehenden Ansprüche, **dadurch gekennzeichnet, dass** es außerdem wenigstens ein Paar umfasst, das eine Nut (91), die in einem der folgenden Elemente verwirklicht ist: gekrümmte Achse (60) und eines der Teile (11-1, 11-2) des länglichen Körpers (11), und eine Rippe (92) komplementär zu der Nut (91) umfasst, wobei die Rippe in dem jeweils Anderen der zwei Elemente verwirklicht ist, derart, dass die Rippe (92) in der Nut (91) gleiten kann.

10. System nach Anspruch 9, **dadurch gekennzeichnet, dass** die Nut (91) und die Rippe (92) gemäß einer Schwalbenschwanzmontage zusammenwirken.

11. System nach einem der vorhergehenden Ansprüche, **dadurch gekennzeichnet, dass** der Führungsstift (50) aus einem Hohlrohr (55) gebildet ist, dass der Druckstift (80) in dem Hohlrohr gleitend montiert ist und dass die Mittel (70) zum Montieren des ersten Endes (61) der gekrümmten Seele (60) in Zusammenwirkung mit dem ersten Ende (51) des Führungsstifts (50) durch Mittel zum Befestigen des ersten Endes (61) der gekrümmten Seele (60) an der Wand des Hohlrohrs und in diese übergehend gebildet sind.

## Claims

1. A system for replacing a disk between two consecutive vertebrae (V1, V2) comprising at least one intervertebral cage (10) and an introducer guide (40) for introducing said intervertebral cage between the two vertebrae, said intervertebral cage (10) being constituted by an oblong body (11) defined between two substantially parallel opposite faces, said oblong body comprising at least two portions (11-1, 11-2) separated by a gap (13), said gap being defined along a curved axis (12) contained in a midplane substantially parallel to the planes of the opposite faces and opening out via an opening (15) at a first end (14) of the oblong body (11) between said two portions (11-1, 11-2) of said oblong body; and the introducer guide (40) being constituted by at least a guide rod (50);
**characterized by** the fact that said introducer guide is constituted besides by:
· a curved longitudinal core (60) of cross-sections that are substantially complementary to the cross-sections of said space (13), so as to enable said curved core to slide relative to the two portions (11-1, 11-2) of said oblong body (11);
· means (70) for mounting a first end (61) of said curved core (60) to co-operate with a first end (51) of said guide rod (50) in such a manner that said curved core (60) projects from said first end (51) of said guide rod (50); and
· a push rod (80) mounted to move relative to said guide rod (50) in such a manner as to be suitable for coming into contact via one of its ends (81) against the first end (14) of the oblong body (11) in order to push said oblong body in translation relative to said curved core (60).

2. A system according to claim 1, **characterized by** the fact that said oblong body (11) is constituted by a single part (111) and that said gap (13) is constituted by a hole formed in said single part, substantially along said curved axis (12).

3. A system according to claim 1, **characterized by** the fact that the single part (111) is substantially in the shape of a kidney bean having opposite faces that are substantially plane.

4. A system according to claim 2 or claim 3, **characterized by** the fact that all of the cross-sections of said curved core (60), as defined in planes perpendicular to said curved axis (12), are substantially mutually identical.

5. A system according to claim 4, **characterized by** the fact that the cross-sections of said curved core (60), defined in planes perpendicular to said curved axis (12), are polygonal in shape.

6. A system according to claim 1, **characterized by** the fact that the two portions (11-1, 11-2) of said oblong body (11) are constituted by two mutually separate parts (211, 212).

7. A system according to claim 6, **characterized by** the fact that the cross-sections of said curved core (60), defined in planes perpendicular to said curved axis (12), are of values that increase substantially continuously from its first end (61) to its free second or other end (62) opposite from the first.

8. A system according to claim 6, **characterized by** the fact that all of the cross-sections of said curved core (60), as defined in planes perpendicular to said curved axis (12), are substantially mutually identical.

9. A system according to any preceding claim, **characterized by** the fact that it further includes at least one groove-and-rib pair comprising both a groove (91) formed in one of the following two elements: the curved core (60) and one of the portions (11-1, 11-2) of said oblong body (11), and also a rib (92) complementary to said groove (91), said rib being made on the other one of the two elements in such a manner that said rib (92) is suitable for sliding in said groove (91).

10. A system according to claim 9, **characterized by** the fact that said groove (91) and said rib (92) co-operate by dovetail mounting.

11. A system according to any preceding claim, **characterized by** the fact that said guide rod (50) is constituted by a hollow tube (55), that the push rod (80) is slidably mounted in said hollow tube, and that the means (70) for mounting the first end (61) of said curved core (60) in co-operation with the first end (51) of said guide rod (50) are constituted by fastener means for fastening the first end (61) of said curved core (60) on the wall of said hollow tube and in continuity therewith.
